# EUROPEAN PATENT SPECIFICATION

(11) **EP 4 574 847 B1**
(45) Date of publication and mention of the grant of the patent: **29.04.2026**
(21) Application number: 24211910.5
(22) Date of filing: 08.11.2024
(51) Int. Cl.: C08B 37/00, A61K 31/715, A61K 31/716, A61K 31/738, C08K 5/00

(54) **GLUCOGALACTAN-BASED INJECTABLE SUBCUTANEOUS FILLING HYDROGEL, AND PREPARATION METHOD AND USE THEREOF**
INJIZIERBARES HYDROGEL MIT SUBKUTANER FÜLLUNG AUF GLUCOGALAKTANBASIS SOWIE HERSTELLUNGSVERFAHREN UND VERWENDUNG DAVON
HYDROGEL INJECTABLE À BASE DE GLUCOGALACTANE DE REMPLISSAGE SOUS-CUTANÉ, SON PROCÉDÉ DE PRÉPARATION ET SON UTILISATION

(30) Priority: 22.12.2023 CN 202311782845
(43) Date of publication of application: 25.06.2025
(73) Proprietor: Nanjing Southern Element Biotechnology Co., Ltd., Nanjing City, Jiangsu 210031 (CN)
(72) Inventor: LI, Bing, Nanjing, 210031 (CN); WANG, Ni, Nanjing, 210031 (CN); ZHANG, Jianfa, Nanjing, 210031 (CN)
(74) Representative: Calysta NV

(56) References cited:
- CORRADI DA SILVA MARIA L ET AL: "Glucogalactan: A polysaccharide isolated from the cell-wall ofVerticillium Lecanii", CARBOHYDRATE POLYMERS, APPLIED SCIENCE PUBLISHERS , LTD BARKING, GB, vol. 98, no. 2, 3 August 2013 (2013-08-03), pages 1353 - 1359, XP028718930, ISSN: 0144-8617, DOI: 10.1016/J.CARBPOL.2013.07.075
- SABATINO MARIA ANTONIETTA ET AL: "Development of injectable and durable kefiran hydro-alcoholic gels", INTERNATIONAL JOURNAL OF BIOLOGICAL MACROMOLECULES, ELSEVIER BV, NL, vol. 149, 25 January 2020 (2020-01-25), pages 309 - 319, XP086081674, ISSN: 0141-8130, [retrieved on 20200125], DOI: 10.1016/J.IJBIOMAC.2020.01.244
- RADHOUANI HAJER ET AL: "Synthesis and Characterization of Biocompatible Methacrylated Kefiran Hydrogels: Towards Tissue Engineering Applications", POLYMERS, vol. 13, no. 8, 20 April 2021 (2021-04-20), CH, pages 1 - 16, XP093116510, ISSN: 2073-4360, DOI: 10.3390/polym13081342

## Description

### TECHNICAL FIELD

The present disclosure belongs to the technical field of biomaterials and hydrogels, and in particular relates to a glucogalactan-based injectable subcutaneous filling hydrogel, and a preparation method and use thereof.

### BACKGROUND

Tumor resection, traumatic injury, lipoatrophy, congenital defects, and aging can lead to loss of the normal structure of soft tissues such as subcutaneous fat, collagen, and muscle. In order to repair defects, many artificial fillers have been widely used in clinical soft tissue filling.

An ideal soft tissue filler should have desirable biocompatibility, flexibility, and biostability. Polysaccharide-based hydrogels are abundant in natural sources. Hyaluronic acid is an important component of the natural extracellular matrix and dominates the health and cosmetics market. However, the hyaluronic acid is easily degraded *in vivo* due to hyaluronidase. Existing commercial hyaluronic acid-based hydrogels are made by combining the hyaluronic acid with a cross-linking agent under certain conditions. Although such hydrogels have a longer degradation time, there is still a demand of repeated injection within one year, increasing the pain of patients.

The applicant has disclosed in patent CN111286466A a strain of *Agrobacterium* and a method for producing and preparing an anti-inflammatory exopolysaccharide produced by the *Agrobacterium.* At the same time, a paper published by the applicant has provided structural analysis of the exopolysaccharide (Anti-tumor activity and immunogenicity of a succinoglycan riclin. Carbohydr Polym. 2021 Mar 1; 255: 117370.). Glucogalactan is a polysaccharide prepared by treating the exopolysaccharide with alkali heat and removing a succinyl group.

### SUMMARY

In order to solve the problems in the prior art, an objective of the present disclosure is to provide an injectable polysaccharide hydrogel with high safety, excellent mechanical properties, and desirable biocompatibility to meet the demands of subcutaneous filling.

To achieve the above objective, the present disclosure provides the following technical solutions:

The present disclosure provides a preparation method of a glucogalactan-based injectable subcutaneous filling hydrogel, including the following steps:
(1) treating a succinoglycan Riclin solution by an alkaline heat method to obtain pure glucogalactan;
(2) dissolving the pure glucogalactan in a NaOH aqueous solution and then stirring at a room temperature to obtain a transparent glucogalactan solution;
(3) adding a cross-linking agent into the transparent glucogalactan solution and then stirring at a room temperature to allow cross-linking to obtain a glucogalactan hydrogel;
(4) neutralizing the glucogalactan hydrogel with an acidic solution, and then washing with neutral deionized water 8 to 15 times to obtain a neutral glucogalactan hydrogel; and
(5) swelling the neutral glucogalactan hydrogel to a certain volume and then sterilizing at a high temperature to obtain a sterile neutral glucogalactan hydrogel.

Further, the succinoglycan Riclin solution has a concentration of 0.5 wt% to 2 wt%, NaOH has a concentration of 0.1 wt%, and the treating is conducted at 105°C for 10 min in step (1).

Further, the transparent glucogalactan solution has a concentration of 8 wt% to 10 wt% and the NaOH aqueous solution has a concentration of 0.1 M to 0.2 M in step (2).

Further, poly(ethylene glycol) diglycidyl ether (PEGDE) is used as the cross-linking agent, and the transparent glucogalactan solution and the cross-linking agent are at a volume ratio of 1:0.036 to 1:0.108 in step (3).

Further, the cross-linking in step (3) is conducted at 30°C to 60°C for 8 h to 16 h.

Further, the acidic solution in step (4) is an HCl solution of 0.1 M to 0.2 M.

Further, the neutral glucogalactan hydrogel in step (4) has 20 mg/mL of the glucogalactan.

Further, the sterilizing in step (5) is conducted at 121°C for 20 min.

Another objective of the present disclosure is to provide a glucogalactan-based injectable subcutaneous filling hydrogel that is maintained subcutaneously for a long time.

Another objective of the present disclosure is to provide a glucogalactan hydrogel according to the present invention for use in preparation of a subcutaneous soft tissue filling material or a joint cavity lubricating fluid.

The present disclosure has the following beneficial effects:

Polyethylene glycol (PEG) is a non-toxic and FDA-approved amphiphilic polyether compound. PEG with a low molecular weight (Mw<1,000) can be degraded and cleared *in vivo.* A novel polysaccharide-based hydrogel is prepared by cross-linking of PEGDE and glucogalactan under alkaline conditions. The results show that glucogalactan hydrogel is a promising biomaterial for soft tissue augmentation.
(1) The present disclosure provides a novel microbial polysaccharide-based chemically cross-linked injectable hydrogel. The microbial polysaccharide-based hydrogel has desirable biocompatibility, superior mechanical properties, and long *in vivo* duration, showing a great potential in cosmetic medicine.
(2) In the present disclosure, the preparation method has the advantages of simple process, mild reaction conditions, and easy synthesis, and is suitable for popularization and use.

### BRIEF DESCRIPTION OF THE DRAWINGS

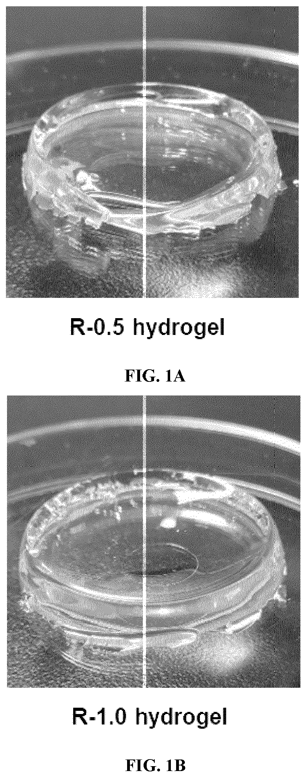

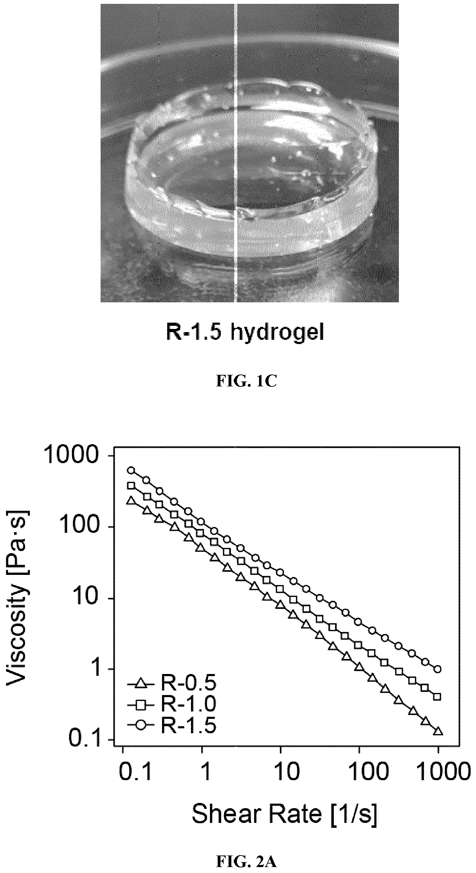

FIGS. 1A-1C show the gelation appearance of glucogalactan hydrogel;

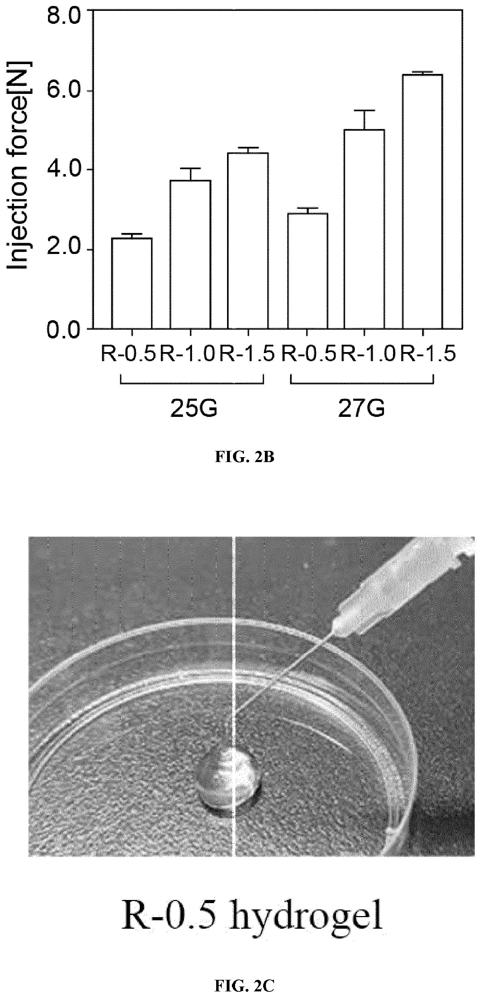

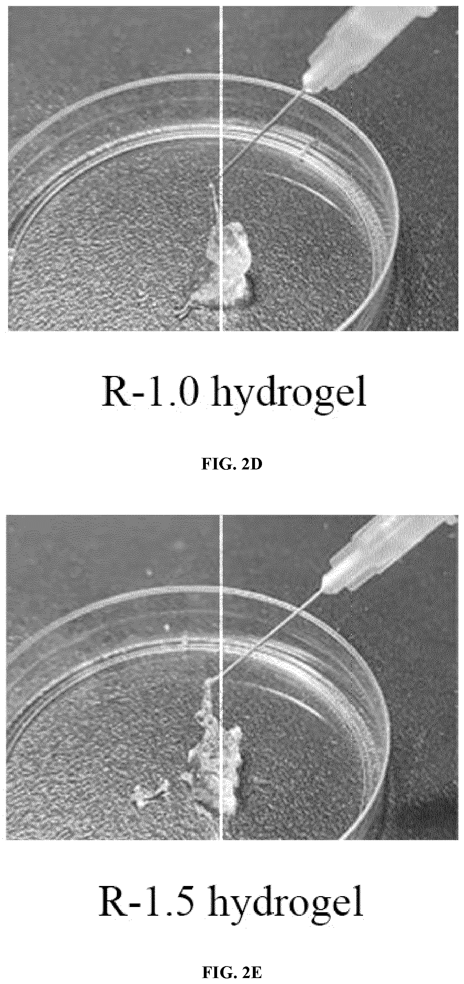

FIGS. 2A-2E show a schematic diagram of injectability of the glucogalactan hydrogel, where FIGS. 2A-2B are a shear rate scanning curve of the glucogalactan hydrogel and an injection force diagram of the glucogalactan hydrogel injected through 25-gauge and 27-gauge needles; FIGS. 2C-2E are schematic diagrams of the injection of R-0.5 hydrogel, R-1.0 hydrogel, and R-1.5 hydrogel through a 27-gauge needle;

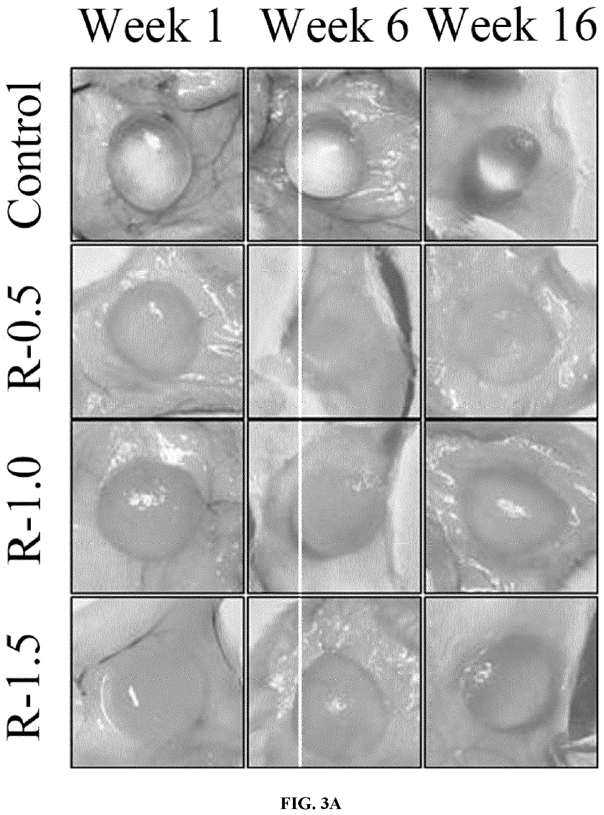

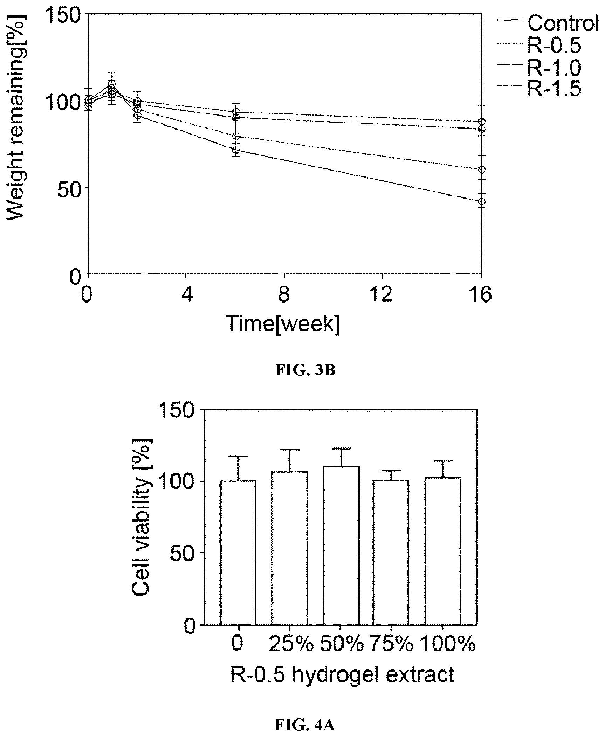

FIGS. 3A-3B show an effect of injecting the glucogalactan hydrogel into the back of mice, where FIG. 3A is a macroscopic photo of the glucogalactan hydrogel after cutting open the back skin of mice at the 1st, 6th, and 16th week after surgery; FIG. 3B shows a degradation trend of the hydrogel in mice; and

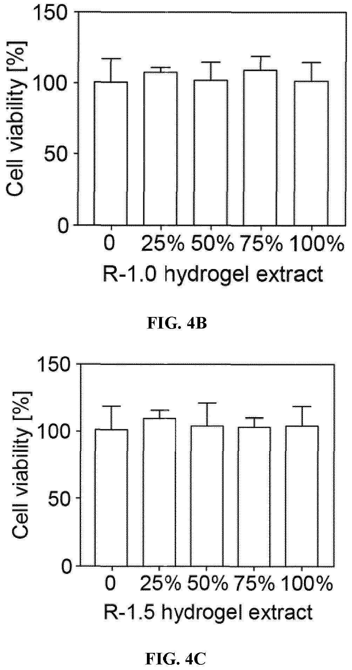

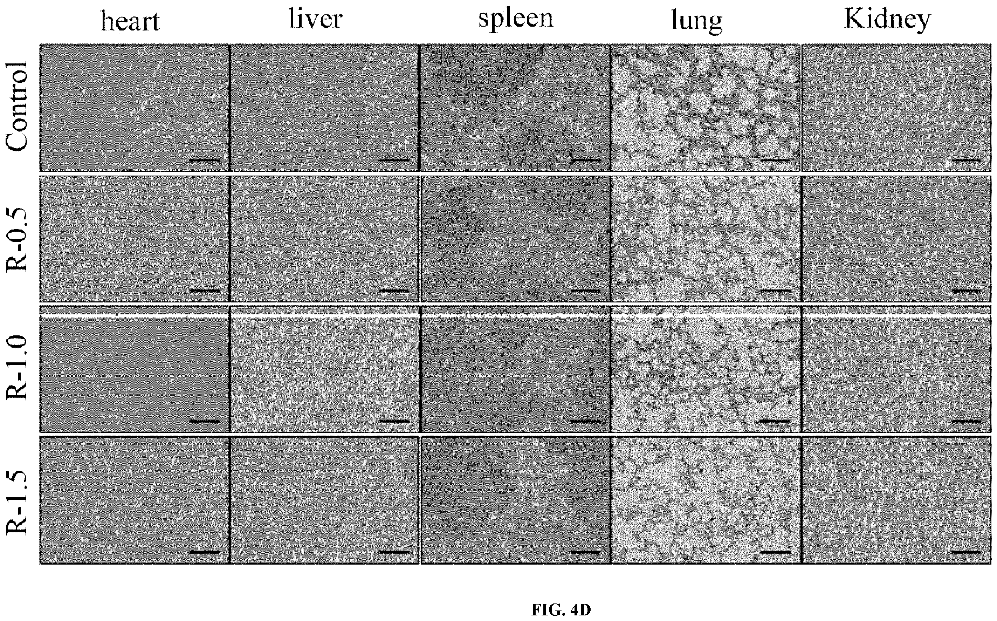

FIGS. 4A-4D show a biosafety effect of the glucogalactan hydrogel, where FIGS. 4A-4C are survival rate of L929 cells cultured with glucogalactan hydrogel extracts of different concentrations for 24 h; FIG. 4D is the histological evaluation of major organs (heart, liver, spleen, lung, and kidney) 1 week after subcutaneous injection of the glucogalactan hydrogel.

### DETAILED DESCRIPTION OF THE EMBODIMENTS

Unless otherwise specified, all technical and scientific terms used in the present disclosure have the same meaning as commonly understood by one of ordinary skill in the technical field to which the present disclosure belongs. Unless otherwise specified, the reagents or materials used in the following examples can be purchased commercially or synthesized by referring to existing methods.

The foregoing is detailed description of the preferred specific examples of the present disclosure. Apparently, the described examples are merely a part rather than all of the examples of the present disclosure. It should be understood that a person of ordinary skill in the art can make various modifications and variations according to the concept of the present disclosure without creative efforts. Therefore, all technical solutions that a person skilled in the art can obtain based on the prior art through logical analysis, reasoning, or finite experiments according to the concept of the present disclosure shall fall within the protection scope defined by the appended claims.

### Example 1

### Preparation of glucogalactan:

(1) A succinoglycan Riclin powder was dissolved in pure water to obtain a Riclin polysaccharide solution with a concentration of 0.5 wt%.
(2) 0.1 wt% NaOH was added into the Riclin polysaccharide solution, mixed well by stirring, and reacted at 105°C for 10 min.
(3) A reacted polysaccharide solution was filtered with a 1 µm fiber membrane, a pH value of an obtained filtrate to neutral, and alcohol was added to obtain a polysaccharide precipitate, namely the glucogalactan; where the glucogalactan had a structural formula as follows:

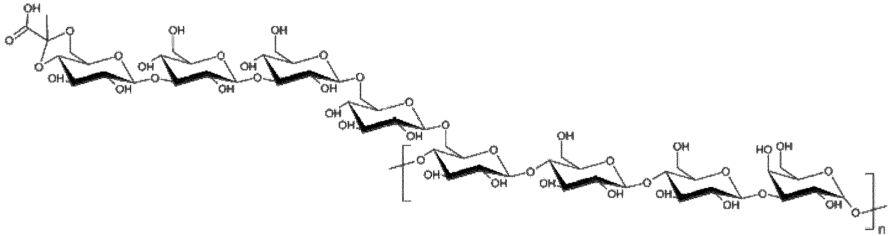

n is 1,000 to 2,000.

### Example 2

Preparation of glucogalactan-based injectable subcutaneous filling hydrogel:
(1) The glucogalactan polysaccharide powder prepared in Example 1 was dissolved in a 0.1 M NaOH aqueous solution at a room temperature to obtain a transparent solution with a sugar concentration of 8 wt%, which was a glucogalactan solution.
(2) A certain amount of PEGDE cross-linking agent was added into the glucogalactan solution, such that the polysaccharide solution and the cross-linking agent were at a volume ratio of 1:0.036, 1:0.072, and 1:0.108. A resulting mixture was mixed uniformly by stirring at a room temperature, and a glucogalactan hydrogel was obtained by cross-linking at 40°C for 8 h.
(3) The glucogalactan hydrogel was neutralized with a 0.1 M HCl solution, and then washed with neutral deionized water 8 to 15 times to obtain a neutral glucogalactan hydrogel with a glucogalactan content of 20 mg/mL.
(4) The neutral glucogalactan hydrogel was sterilized at 121°C under high pressure for 20 min to obtain a sterile neutral glucogalactan hydrogel.

According to the above preparation method, 8 wt% of glucogalactan polysaccharide solution was cross-linked with PEGDE at volume ratios of 1:0.036, 1:0.072, and 1:0.108, and then R-0.5 hydrogel, R-1.0 hydrogel, and R-1.5 hydrogel with a glucogalactan polysaccharide content of 20 mg/mL were obtained after swelling, respectively.

FIGS. 1A-1C showed the appearances of R-0.5 hydrogel, R-1.0 hydrogel, and R-1.5 hydrogel formed by cross-linking of glucogalactan polysaccharide.

### Example 3

Determination of injectability of the glucogalactan-based injectable subcutaneous filling hydrogel:
The R-0.5 hydrogel, R-1.0 hydrogel, and R-1.5 hydrogel were tested in sequence, and the injectability of the glucogalactan hydrogel was characterized by a rheometer. The viscosity of the hydrogels was measured as a function of shear rate (0.1-1000 1/s) in the complex viscosity test. The R-0.5 hydrogel, R-1.0 hydrogel, and R-1.5 hydrogel were loaded into 1 mL syringes using 25-gauge and 27-gauge needles, respectively, for injectability testing.

As shown in FIGS. 2A-2B, the viscosity of the R-0.5 hydrogel, the R-1.0 hydrogel, and the R-1.5 hydrogel rapidly decreased by three orders of magnitude with the increase of the shear rate. Injectability was verified at the macroscopic level.

As shown in FIGS. 2C-2E, the three hydrogels could be smoothly extruded through 25-gauge and 27-gauge needles and integrated together to form a viscous whole gel, thus further intuitively verifying the injectability of the glucogalactan hydrogel.

### Example 4

*In vivo* study of glucogalactan-based injectable subcutaneous filling hydrogel in mice:
During the modeling, the mice were randomly divided into 4 groups, namely R-0.5, R-1.0, R-1.5, and control groups. The back of the mice was depilated, and the glucogalactan hydrogel or commercial hyaluronic acid hydrogel (Bloomage Biotech, BIOHYALUX^{®}, modified sodium hyaluronate gel for injection, production license number: Shandong Food and Drug Administration Machinery Production License No. 20120123) was injected into the back of the mice. The mice were sacrificed at 1 week, 6 weeks, and 16 weeks after hydrogel injection, and the skin tissue and hydrogel in the hydrogel area were cut off to observe the appearance and size of the hydrogel and measure the changes in weight of the hydrogel.

As shown in FIG. 3A, the control group was the commercial hyaluronic acid hydrogel. As the implantation time changed, the appearance and size of the hydrogel in the control group changed significantly due to the presence of hyaluronidase *in vivo.* The shape and size of the glucogalactan hydrogel also changed. The R-0.5 hydrogel was thin and soft, which might be due to weak cross-linking strength and difficulty in maintaining the mechanical properties for a long time under the skin. However, the R-1.0 hydrogel and R-1.5 hydrogel had better maintenance effects.

As shown in FIG. 3B, the commercial hyaluronic acid hydrogel had been degraded to about 50% of its original weight after being *in vivo* for more than 16 weeks. The R-1.0 hydrogel and R-1.5 hydrogel retained more than 80% of their weights and were not easily degraded *in vivo,* among which the R-0.5 hydrogel degraded to about 60% of its original weight due to weaker cross-linking strength.

### Example 5

Safety evaluation of glucogalactan-based injectable subcutaneous filling hydrogel:

### (1) Cytotoxicity test:

In this study, the MTT assay and live/dead imaging were conducted to evaluate the *in vitro* cytotoxicity of glucogalactan and glucogalactan hydrogel. L929 cells were seeded in a 96-well plate and cultured for 24 h. 1 mL of hydrogel was incubated in DMEM for 48 h, a glucogalactan hydrogel extract was prepared at 37°C, and then transferred into a regular DMEM medium containing different concentrations of glucogalactan hydrogel extracts to allow culture for another 24 h. After MTT treatment, cell viability was measured at 570 nm.

### (2) Visceral safety evaluation:

In this study, the *in vivo* degradation and biocompatibility of glucogalactan hydrogels were evaluated by subcutaneous implantation in the back of Balb/c mice. 200 µL of sterile hydrogel was injected subcutaneously into the dorsal flank of the mouse using a 27-gauge needle. Mice were euthanized 7 d after implantation. The main organs (heart, liver, spleen, lung, and kidney) were taken for H&E staining and Masson staining to analyze whether the glucogalactan hydrogel was toxic to the internal organs of mice.

As shown in FIGS. 4A-4C, the cell viability was over 90% in all ranges, indicating that the glucogalactan hydrogel had desirable *in vitro* biocompatibility.

As shown in FIG. 4D, no obvious inflammatory reaction or tissue necrosis was observed from the H&E staining images. This further confirmed the low *in vivo* toxicity of glucogalactan hydrogel and its desirable biocompatibility.

From the above examples, it can be seen that a polysaccharide glucogalactan is dissolved in a NaOH aqueous solution and then mixed evenly by stirring, a proper amount of a cross-linking agent is added and then stirred evenly, and a glucogalactan hydrogel is prepared by a reaction under appropriate conditions. The preparation method has mild conditions, simple process, few reagents, no violent reactions, and low cost, and is easy to achieve industrial production. The polysaccharide hydrogel prepared shows high degradation resistance, excellent mechanical properties, desirable biocompatibility, and long duration *in vivo.* The glucogalactan hydrogel is suitable for scenarios such as subcutaneous filling, wrinkle removal, and joint cavity lubricating fluid replacement.

## Claims

1. A preparation method of a glucogalactan-based injectable subcutaneous filling hydrogel, comprising the following steps:
(1) dissolving glucogalactan in a NaOH aqueous solution and then stirring at a room temperature to obtain a transparent glucogalactan solution;
(2) adding a cross-linking agent into the transparent glucogalactan solution and then stirring at a room temperature to allow cross-linking to obtain a glucogalactan hydrogel;
(3) neutralizing the glucogalactan hydrogel with an acidic solution, and then washing with neutral deionized water 8 to 15 times to obtain a neutral glucogalactan hydrogel; and
(4) swelling the neutral glucogalactan hydrogel to a certain volume and then sterilizing at a high temperature to obtain a sterile neutral glucogalactan hydrogel.

2. The preparation method of a glucogalactan-based injectable subcutaneous filling hydrogel according to claim 1, wherein the glucogalactan in step (1) has a structural formula as follows: 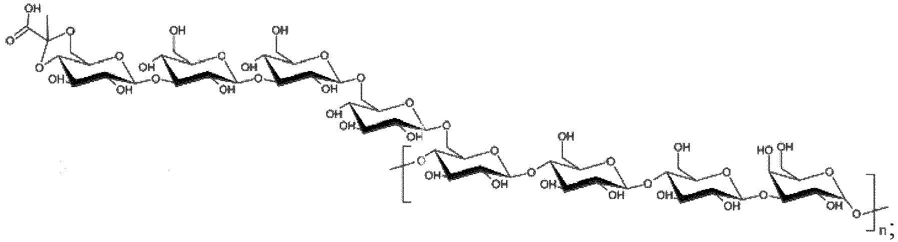 n is 500 to 10,000.

3. The preparation method of a glucogalactan-based injectable subcutaneous filling hydrogel according to claim 1, wherein the transparent glucogalactan solution has a concentration of 8 wt% to 10 wt% and the NaOH aqueous solution has a concentration of 0.1 M to 0.2 M in step (1).

4. The preparation method of a glucogalactan-based injectable subcutaneous filling hydrogel according to claim 1, wherein the transparent glucogalactan solution and the cross-linking agent are at a volume ratio of 1:0.036 to 1:0.108, and the cross-linking agent is one or more selected from the group consisting of carbodiimide, divinyl sulfone, 1,4-butanediol diglycidyl ether (BDDE), and poly(ethylene glycol) diglycidyl ether (PEGDE) in step (2).

5. The preparation method of a glucogalactan-based injectable subcutaneous filling hydrogel according to claim 1, wherein the cross-linking in step (2) is conducted at 30°C to 60°C for 8 h to 16 h.

6. The preparation method of a glucogalactan-based injectable subcutaneous filling hydrogel according to claim 1, wherein the acidic solution in step (3) is an HCl solution of 0.1 M to 0.2 M.

7. The preparation method of a glucogalactan-based injectable subcutaneous filling hydrogel according to claim 1, wherein the neutral glucogalactan hydrogel in step (3) has 20 mg/mL of the glucogalactan.

8. The preparation method of a glucogalactan-based injectable subcutaneous filling hydrogel according to claim 1, wherein the sterilizing in step (4) is conducted at 121°C for 20 min.

9. A glucogalactan hydrogel prepared by the preparation method according to any one of claims 1 to 8.

10. Glucogalactan hydrogel according to claim 9 for use in preparation of a subcutaneous soft tissue filling material or a joint cavity lubricating fluid.

## Patentansprüche

1. Herstellungsverfahren für ein injizierbares subkutanes Füllhydrogel auf Glucogalactanbasis, umfassend die folgenden Schritte:
(1) Auflösen von Glucogalactan in einer wässrigen NaOH-Lösung und anschließendes Rühren bei Raumtemperatur, um eine transparente Glucogalactan-Lösung zu erhalten;
(2) Zusetzen eines Vernetzungsmittels zu der transparenten Glucogalactan-Lösung und anschließendes Rühren bei Raumtemperatur, um eine Vernetzung zu ermöglichen und ein Glucogalactan-Hydrogel zu erhalten;
(3) Neutralisieren des Glucogalactan-Hydrogels mit einer sauren Lösung und anschließendes 8- bis 15-maliges Waschen mit neutralem entionisiertem Wasser, um ein neutrales Glucogalactan-Hydrogel zu erhalten; und
(4) Quellenlassen des neutralen Glucogalactan-Hydrogels auf ein bestimmtes Volumen und anschließendes Sterilisieren bei einer hohen Temperatur, um ein steriles neutrales Glucogalactan-Hydrogel zu erhalten.

2. Herstellungsverfahren für ein injizierbares subkutanes Füllhydrogel auf Glucogalactanbasis nach Anspruch 1, wobei das Glucogalactan in Schritt (1) eine Strukturformel aufweist, wie folgt: 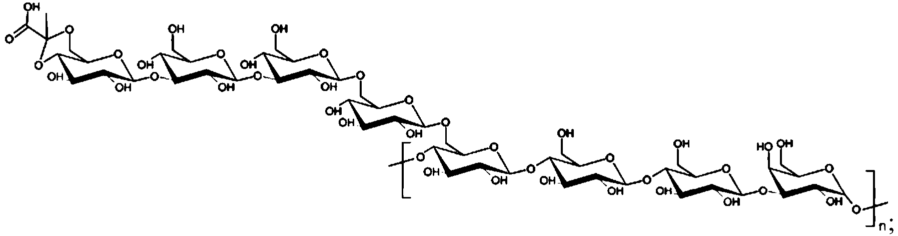 n ist 500 zu 10.000.

3. Herstellungsverfahren für ein injizierbares subkutanes Füllhydrogel auf Glucogalactanbasis nach Anspruch 1, wobei die transparente Glucogalactan-Lösung eine Konzentration von 8 Gew.-% bis 10 Gew.-% und die wässrige NaOH-Lösung eine Konzentration von 0,1 M bis 0,2 M in Schritt (1) aufweist.

4. Herstellungsverfahren für ein injizierbares subkutanes Füllhydrogel auf Glucogalactanbasis nach Anspruch 1, wobei die transparente Glucogalactan-Lösung und das Vernetzungsmittel in einem Volumenverhältnis von 1:0,036 bis 1:0,108 vorliegen und das Vernetzungsmittel eines oder mehrere ausgewählt aus der Gruppe bestehend aus Carbodiimid, Divinylsulfon, 1,4-Butanediol diglycidylether (BDDE) und Poly(ethylenglycol)-diglycidylether (PEGDE) in Schritt (2) ist.

5. Herstellungsverfahren für ein injizierbares subkutanes Füllhydrogel auf Glucogalactanbasis nach Anspruch 1, wobei die Vernetzung in Schritt (2) bei 30 °C bis 60 °C für 8 Stunden bis 16 Stunden ausgeführt wird.

6. Herstellungsverfahren für ein injizierbares subkutanes Füllhydrogel auf Glucogalactanbasis nach Anspruch 1, wobei die saure Lösung in Schritt (3) eine HCL-Lösung von 0,1 M bis 0,2 M ist.

7. Herstellungsverfahren für ein injizierbares subkutanes Füllhydrogel auf Glucogalactanbasis nach Anspruch 1, wobei das neutrale Glucogalactan-Hydrogel in Schritt (3) 20 mg/ml Glucogalactan aufweist.

8. Herstellungsverfahren für ein injizierbares subkutanes Füllhydrogel auf Glucogalactanbasis nach Anspruch 1, wobei die Vernetzung in Schritt (4) bei 121 °C für 20 min ausgeführt wird.

9. Glucogalactan-Hydrogel, das nach dem Herstellungsverfahren nach einem der Ansprüche 1 bis 8 hergestellt wird.

10. Glucogalactan-Hydrogel nach Anspruch 9 für die Verwendung zur Herstellung eines subkutanen Weichgewebefüllmaterials oder einer Gelenkspalt-Schmierflüssigkeit.

## Revendications

1. Procédé de préparation d'un hydrogel de comblement sous-cutané injectable à base de glucogalactane, comprenant les étapes suivantes :
(1) dissoudre du glucogalactane dans une solution aqueuse de NaOH, puis agiter à température ambiante afin d'obtenir une solution de glucogalactane transparente ;
(2) ajouter un agent de réticulation à la solution de glucogalactane transparente, puis agiter à température ambiante pour permettre la réticulation afin d'obtenir un hydrogel de glucogalactane ;
(3) neutraliser l'hydrogel de glucogalactane avec une solution acide, puis laver avec de l'eau désionisée neutre 8 à 15 fois afin d'obtenir un hydrogel de glucogalactane neutre ; et
(4) faire gonfler l'hydrogel de glucogalactane neutre jusqu'à un certain volume, puis stériliser à haute température afin d'obtenir un hydrogel de glucogalactane neutre stérile.

2. Procédé de préparation d'un hydrogel de comblement sous-cutané injectable à base de glucogalactane selon la revendication 1, dans lequel le glucogalactane à l'étape (1) a une formule structurelle comme suit : 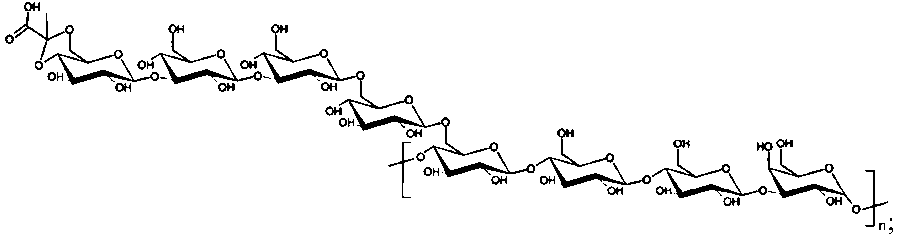 n vaut 500 à 10 000.

3. Procédé de préparation d'un hydrogel de comblement sous-cutané injectable à base de glucogalactane selon la revendication 1, dans lequel la solution de glucogalactane transparente a une concentration de 8 % en poids à 10 % en poids et la solution aqueuse de NaOH a une concentration de 0,1 M à 0,2 M à l'étape (1).

4. Procédé de préparation d'un hydrogel de comblement sous-cutané injectable à base de glucogalactane selon la revendication 1, dans lequel la solution de glucogalactane transparente et l'agent de réticulation sont à un rapport volumique de 1:0,036 à 1:0,108, et l'agent de réticulation est un ou plusieurs éléments choisis dans le groupe constitué par le carbodiimide, la divinyl sulfone, le diglycidyl éther de 1,4-butanediol (BDDE), et le diglycidyl éther de poly(éthylène glycol) (PEGDE) à l'étape (2).

5. Procédé de préparation d'un hydrogel de comblement sous-cutané injectable à base de glucogalactane selon la revendication 1, dans lequel la réticulation à l'étape (2) est menée à 30 °C à 60 °C pendant 8 h à 16 h.

6. Procédé de préparation d'un hydrogel de comblement sous-cutané injectable à base de glucogalactane selon la revendication 1, dans lequel la solution acide à l'étape (3) est une solution de HCl de 0,1 M à 0,2 M.

7. Procédé de préparation d'un hydrogel de comblement sous-cutané injectable à base de glucogalactane selon la revendication 1, dans lequel l'hydrogel de glucogalactane neutre à l'étape (3) a 20 mg/mL du glucogalactane.

8. Procédé de préparation d'un hydrogel de comblement sous-cutané injectable à base de glucogalactane selon la revendication 1, dans lequel la stérilisation à l'étape (4) est menée à 121 °C pendant 20 min.

9. Hydrogel de glucogalactane préparé par le procédé de préparation selon l'une quelconque des revendications 1 à 8.

10. Hydrogel de glucogalactane selon la revendication 9 pour une utilisation dans la préparation d'un matériau de comblement de tissu mou sous-cutané ou d'un fluide lubrifiant de cavité articulaire.
